# EUROPEAN PATENT APPLICATION

(11) **EP 4 534 670 A1**
(43) Date of publication of application: **09.04.2025**
(21) Application number: 23816260.6
(22) Date of filing: 16.05.2023
(51) Int. Cl.: C12N 15/113, C12N 15/63, A61K 48/00, A61K 31/7088, A61P 35/00

(54) **COMPOSITION FOR REGULATING SPLICING OF POLYBROMO 1 GENE COMPRISING SPLICE-SWITCHING OLIGONUCLEOTIDE AS ACTIVE INGREDIENT**

(30) Priority: 03.06.2022 KR 20220068085
(71) Applicant: The Industry & Academic Cooperation in Chungnam National University (IAC), Daejeon 34134 (KR); Korea Research Institute of Chemical Technology, Daejeon 34114 (KR)
(72) Inventor: KIM, Keekwang, Cheongyang-gun, Chungcheongnam-do 33361 (KR); CHO, Namjoon, Daejeon 35398 (KR); KIM, Eun-Mi, Daejeon 34974 (KR)
(74) Representative: Cabinet Nony
(86) International application number: PCT/KR2023/006600
(87) International publication number: WO 2023/234597

(57) **Abstract**

The present invention relates to a composition for regulating splicing of Polybromo 1 (PBRM1) gene comprising a splicing-switch oligonucleotide as an effective component, and the splicing-switch oligonucleotide as an effective component of the present invention exhibits the effect of regulating the splicing of PBRM1 (Polybromo 1) gene and also, when exon 27 is skipped in cancer cells through the splicing regulation, enhancing cancer cell death through activation of NK92 cells.

## Description

### TECHNICAL FIELD

The present invention relates to a composition for regulating splicing of Polybromo 1 gene comprising a splicing-switch oligonucleotide as an effective component.

### BACKGROUND ART

PBRM1 (Polybromo 1) is a constitutional component of the tumor-suppressing protein complex PBAF (SWI/SNF-B). Loss of PBRM1 function due to mutations leads to cancer development through mechanisms such as enhanced immune evasion, promoted expression of oncogene, or accumulation of DNA damage. For instance, mutations in the PBRM1 gene are found in 39% of patients with clear cell renal cell carcinoma (ccRCC), a cancer that accounts for the majority of kidney cancer cases. Based on data from The Cancer Genome Atlas (TCGA), cancer-tissue-specific alternative splicing of PBRM1 has been examined, and it is known that an increase in exon 27 inclusion in PBRM1 is found in most cancer tissues.

Splicing is a process of modifying mRNA by removing introns, which do not carry genetic information, from precursor mRNA (Pre-mRNA), and joining only the exons, which contain genetic information, to yield translation into a single polypeptide chain. It is an essential step in gene expression regulation in eukaryotic organisms. Alternative splicing is a process that generates mRNA through specific combinations of various exons, allowing a single gene to produce multiple types of mRNA. It is known that this process enables the production of diverse proteins without expanding the genome, thus contributing to cellular diversity. However, alternative splicing can also lead to the formation of non-productive mRNA transcripts, which can affect protein expression.

Therapeutic agents targeting the alternative splicing of gene-encoded pre-mRNA can potentially increase the expression level of functional proteins or inhibit abnormal protein expression in patients. Those therapeutic agents can be used for treating clinical conditions benefiting from increased protein expression or addressing diseases that are caused by altered expression level of proteins.

For example, Korean Patent Application Publication No. 2021-0042123 discloses "Alternative splicing regulation of gene expression and therapeutic methods", and Korean Patent Application Publication No. 2010-0101050 discloses "Splice switching oligomers for TNF superfamily receptors and their use in treatment of disease". However, there has been no disclosure relating to "Composition for regulating splicing of Polybromo 1 gene comprising splicing-switch oligonucleotide as an effective component" described in the present invention.

### DETAILED DESCRIPTION OF INVENTION

### TECHNICAL PROBLEMS TO BE SOLVED

The present invention is devised in view of the need described above, and provided by the present invention is a composition for regulating splicing of Polybromo 1 (PBRM1) gene comprising a splicing-switch oligonucleotide as an effective component. It has been found that, when splicing of PBRM1 gene is regulated by utilizing the splicing-switch oligonucleotide, a key component of the present invention, and exon 27 is skipped in cancer cells based on such splicing regulation, the anti-cancer effects of immune cells, including natural killer cells and T cells, are enhanced, and the present invention is completed accordingly.

### TECHNICAL MEANS FOR SOLVING THE PROBLEMS

To achieve the object described in the above, the present invention provides a composition for regulating splicing of PBRM1 (Polybromo 1) gene comprising, as an effective component, a splicing-switch oligonucleotide (SSO) that selectively skips exon 27 of PBRM1 (Polybromo 1) pre-mRNA.

The present invention further provides a pharmaceutical composition for preventing or treating cancer comprising, as an effective component, a splicing-switch oligonucleotide (SSO) that selectively skips exon 27 of PBRM1 (Polybromo 1) pre-mRNA.

The present invention still further provides a method for regulating expression of PBRM1 (Polybromo 1) protein in mammalian cells comprising transducing mammalian cells with a splicing-switch oligonucleotide (SSO) that selectively skips exon 27 of PBRM1 (Polybromo 1) pre-mRNA to induce splicing that selectively skips exon 27 of PBRM1 (Polybromo 1) pre-mRNA through complementary binding between the SSO and PBRM1 (Polybromo 1) pre-mRNA.

### ADVANTAGEOUS EFFECT OF INVENTION

The present invention relates to a composition for regulating splicing of Polybromo 1 gene comprising a splicing-switch oligonucleotide as an effective component. Specifically, the splicing-switch oligonucleotide as an effective component of the present invention exhibits the effect of regulating the splicing of PBRM1 (Polybromo 1) gene and also, when exon 27 is skipped in cancer cells through the splicing regulation, enhancing cancer cell death through activation of immune cells including natural killer cells and T cells.

### BRIEF DESCRIPTION OF THE DRAWINGS

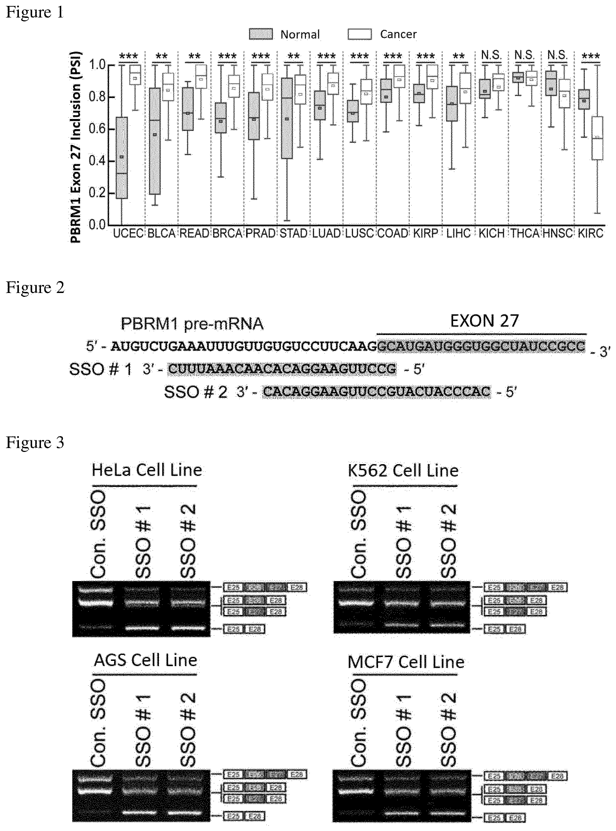

Figure 1 illustrates the result of determining a change in alternative splicing of exon 27 of PBRM1 in cancer tissues compared to normal tissues, in which the determination was made based on analyses using the TCGA cancer database. Specific cancer types include uterine corpus endometrial carcinoma (UCEC), bladder cancer (BLCA), rectal adenocarcinoma (READ), breast cancer (BRCA), prostate adenocarcinoma (PRAD), stomach adenocarcinoma (STAD), lung adenocarcinoma (LUAD), lung squamous cell carcinoma (LUSC), colon adenocarcinoma (COAD), kidney renal papillary cell carcinoma (KIRP), liver cancer (LIHC), kidney chromophobe (KICH), thyroid carcinoma (THCA), head and neck cancer (HNSC), and kidney clear cell carcinoma (KIRC). ** and *** denote statistically significant increases or decreases in PBRM1 exon 27 inclusion in cancer tissues compared to normal tissues, in which ** indicates p<0.01, *** indicates p<0.001, and N.S. (not significant) indicates no statistically significant difference.
Figure 2 is a schematic diagram of two synthetic splicing-switch oligonucleotides (SSOs) designed such that they can bind to PBRM1 pre-mRNA to induce a change in the alternative splicing in exon 27.
Figure 3 shows the result of reverse transcription-polymerase chain reaction (RT-PCR) for determining a change in alternative splicing of PBRM1 exon 27 upon SSO treatment in various cancer cell lines, including HeLa (cervical cancer cell line), K562 (leukemia cell line), AGS (gastric cancer cell line), and MCF7 (breast cancer cell line).

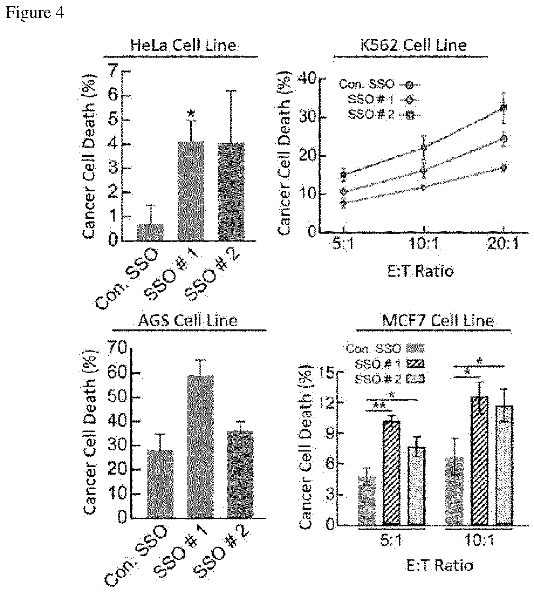

Figure 4 illustrates the result demonstrating that natural killer cells (NK92, Effector Cell: E) enhance the death of cancer cells (Target Cell: T) with induced alternative splicing in exon 27 of PBRM1 caused by SSO treatment. * and ** indicate statistically significant increases in cancer cell death in the test groups of the present invention which have been treated with the SSOs of the present invention (i.e., transduced with SSO#1 or SSO#2 to induce alternative splicing for exon 27 skipping) compared to the control group (Con. SSO), in which * denotes p<0.05, and ** denotes p<0.01.

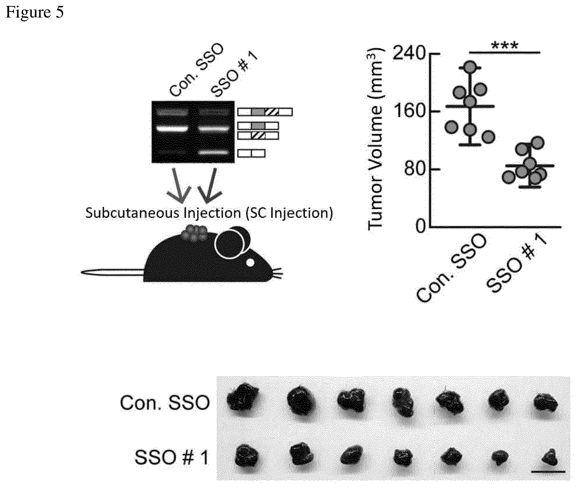

Figure 5 shows the result of measuirng the tumor size on day 9 after subcutaneous injection of melanoma cells treated with the SSO of the present invention, which induces alternative splicing of PBRM1 exon 27, into a mouse. *** indicates a statistically significant decrease in tumor size in the test group which has been subcutaneously injected with melanoma cells treated with the SSO#1 of the present invention compared to the control group (Con. SSO), with p<0.001.

### BEST EMBODIMENT(S) FOR CARRYING OUT INVENTION

The present invention relates to a composition for regulating splicing of PBRM1 (Polybromo 1) gene comprising, as an effective component, a splicing-switch oligonucleotide (SSO) that selectively skips exon 27 of PBRM1 (Polybromo 1) pre-mRNA.

The splicing-switch oligonucleotide (SSO) preferably includes a sequence complementary to exon 27 of PBRM1 (Polybromo 1) pre-mRNA, and more preferably an oligonucleotide sequence consisting of the nucleotide sequence of SEQ ID NO: 1 or SEQ ID NO: 2, although it is not limited thereto.

The splicing-switch oligonucleotide (SSO) is characterized in that it selectively skips exon 27 by complementary binding to PBRM1 (Polybromo 1) pre-mRNA, thereby inhibiting the immune evasion (immune tolerance/immune checkpoint) mechanism in cancer cells.

The present invention further relates to a pharmaceutical composition for preventing or treating cancer comprising, as an effective component, a splicing-switch oligonucleotide (SSO) that selectively skips exon 27 of PBRM1 (Polybromo 1) pre-mRNA.

The splicing-switch oligonucleotide (SSO) that selectively skips exon 27 of PBRM1 (Polybromo 1) pre-mRNA is the same as described in the above.

The effective component may have one or both ends fused to a carrier, either directly or via a linker, or it may preferably include an additional gene carrier besides the effective component, although it is not limited thereto. The term "gene carrier" has the same meaning as the system that assists in the transport of genes into cells, facilitating gene transduction (insertion into cells) or intracellular gene diffusion (spreading within cells).

The gene carrier may preferably be a plasmid; a viral vector; naked recombinant DNA molecules; or a liposome or a niosome that includes the aforementioned naked recombinant DNA molecules or plasmids, although it is not limited thereto.

The cancer may preferably be any one selected from uterine corpus endometrial carcinoma (UCEC), bladder cancer (BLCA), rectal adenocarcinoma (READ), breast cancer (BRCA), prostate adenocarcinoma (PRAD), stomach adenocarcinoma (STAD), lung adenocarcinoma (LUAD), lung squamous cell carcinoma (LUSC), colon adenocarcinoma (COAD), kidney renal papillary cell carcinoma (KIRP), and liver hepatocellular carcinoma (LIHC) although it is not limited thereto.

The pharmaceutical composition of the present invention may include a "pharmaceutically acceptable vehicle", in addition to the component. The term "pharmaceutically effective amount" refers to an amount sufficient to achieve the desired efficacy or activity of the aforementioned effective component.

The pharmaceutical composition of the present invention is preferably administered parenterally, for example, via intravenous, subcutaneous, or topical administration.

The appropriate dosage of the pharmaceutical composition of the present invention may vary based on factors such as formulation method, administration route, age, weight, or sex of a patient, severity of disease symptoms, diet, administration time, administration route, excretion rate, and sensitivity to response. Typically, a skilled physician can easily determine and prescribe an effective dosage for the intended treatment. Generally, the daily dosage of the pharmaceutical composition of the present invention ranges from 0.0001 to 100 mg/kg.

The pharmaceutical composition of the present invention can be formulated using pharmaceutically acceptable vehicles and/or excipients according to methods that can be easily carried out by those skilled in the art to which the present invention pertains. It can be manufactured in unit dosage forms or packaged in multi-dose containers.

The present invention still further relates to a method for regulating expression of PBRM1 (Polybromo 1) protein in mammalian cells comprising transducing mammalian cells with a splicing-switch oligonucleotide (SSO) that selectively skips exon 27 of PBRM1 (Polybromo 1) pre-mRNA to induce splicing that selectively skips exon 27 of PBRM1 (Polybromo 1) pre-mRNA through complementary binding between the SSO and PBRM1 (Polybromo 1) pre-mRNA.

Hereinbelow, the present invention is explained in greater detail in view of Examples. However, the following Examples are given only for more specific explanation of the present invention and it is evident for a person who has common knowledge in the pertinent art that the scope of the present invention is not limited by them.

### EXAMPLES

### Example 1. Analysis of TCGA Cancer Database

Change in alternative splicing of PBRM1 exon 27 was determined in normal tissues and 15 different types of cancer tissues.

As shown in Figure 1, a statistically significant increase in formation of the inclusion of PBRM1 exon 27 was observed in cancer tissues compared to normal tissues for the following types of cancer: uterine corpus endometrial carcinoma (UCEC), bladder cancer (BLCA), rectal adenocarcinoma (READ), breast cancer (BRCA), prostate adenocarcinoma (PRAD), stomach adenocarcinoma (STAD), lung adenocarcinoma (LUAD), lung squamous cell carcinoma (LUSC), colon adenocarcinoma (COAD), kidney renal papillary cell carcinoma (KIRP), and liver hepatocellular carcinoma (LIHC).

### Example 2. Cell Culture

HeLa, MCF7, AGS, K562, A549, and HCT116 cells were cultured in DMEM (Dulbecco's Modified Eagle's Medium) supplemented with 10% fetal bovine serum and 1% penicillin/streptomycin.

NK92 cells were cultured in α-MEM (Minimum Essential Medium) supplemented with 12.5% fetal bovine serum, 12.5% horse serum, 100 µM 2-mercaptoethanol, 200 µM inositol, 20 µM folic acid, 200 U/mL IL-2, and 1% penicillin/streptomycin.

### Example 3. Transduction with SSO (splicing-switch oligonucleotide)

Splicing-switch oligonucleotides (SSOs) were synthesized using a phosphorothioate (PS) backbone with a 2'-O-methyl (2'OMe) modification at the 2' sugar position.

The PBRM1 SSO (Polybromo 1 splicing-switch oligonucleotide) was designed to include the sequences of 5'-GCC UUG AAG GAC ACA AAU UUC-3' (SSO #1; SEQ ID NO: 1) and 5'-CAC CCA UCA UGC CUU GAA GGA CAC-3' (SSO #2; SEQ ID NO: 2) that complementarily bind to the 3' splice site of PBRM1 exon 27. Control SSO was synthesized using the sequence 5'-UGC AUU CGC CCU CUU AAU GGG GA-3' (SEQ ID NO: 3) (Figure 2).

To deliver the SSO to cells, 3×10⁵ cells were plated in each well of a 6-well plate, and transduction with 1 µg of SSO was carried out by using PolyMag reagent (OZ Bioscience, CA, USA) according to the manufacturer's instructions.

### Example 4. RNA Separation and Determination of Change in PBRM1 Exon 27 Splicing in SSO-Transduced Cells

Using the GeneAll Hybrid-R^{™} RNA extraction kit (GeneAll, Seoul, Korea), total RNA was extracted from cervical cancer (HeLa), leukemia (K562), gastric cancer (AGS), and breast cancer (MCF7) cells that have been transduced with the splicing-switch oligonucleotides (SSOs) for 24 hours. For reverse transcription-polymerase chain reaction (RT-PCR), 1 µg of total RNA, random hexamers, M-MLV reverse transcriptase (Promega, WI, USA), RNase inhibitor (Enzynomics, Korea), and GoldHotStart Taq PCR master mix (Bioneer, Korea) were used. After that, any change in the PBRM1 exon 27 splicing was determined through agarose gel electrophoresis. In the RT-PCR reaction, the primers used were 5'-GTA GCC CAC CAA GAC-3' (forward; SEQ ID NO: 4) and 5'-GCT GGA GTC ACC ATA GG-3' (reverse; SEQ ID NO: 5).

As shown in Figure 3, the results indicated that exon 27 skipping was induced in cancer cells which had been transduced with SSO #1 or SSO #2 according to the present invention.

### Example 5. Analysis of Cancer Cell Killing Activity of Natural Killer Cells (NK cells)

5×10³ Cancer cells (cervical cancer HeLa, leukemia K562, gastric cancer AGS, and breast cancer MCF7), which had been transduced with SSO #1 or SSO #2 for 24 hours, were trypsinized for 24 hours. The cells were then mixed with natural killer (NK92) cells in various ratios (effector cell/target cell ratios of 5:1, 10:1, or 20:1) in ultra-low attachment 96-well plates (Corning, USA) and then co-cultured at 37°C for 7 hours.

Cytotoxicity was examined using the CyQUANT^{™} LDH Cytotoxicity Assay Kit (Thermo Fisher Scientific, USA), which measures the secretion amount of lactate dehydrogenase (LDH).

The results, as presented in Figure 4, demonstrated that cancer cell death by the natural killer cells was promoted in cervical cancer (HeLa), leukemia (K562), gastric cancer (AGS), and breast cancer (MCF7) cells which had been transduced with SSO #1 or SSO #2.

### Example 6. Anti-Cancer Effect in Animal Model

A 6-week-old male C57BL/6J mouse was subcutaneously injected with B 16F10 melanoma cells transduced with PBRM1 SSO (n=7). The animal was sacrificed on day 9 to measure tumor size.

The results indicated that the size of tumors (i.e., melanoma) transduced with SSO #1 of the present invention was significantly reduced compared to that of the SSO-transduced melanoma cells (Figure 5).

## Claims

1. A composition for regulating splicing of PBRM1 (Polybromo 1) gene comprising, as an effective component, a splicing-switch oligonucleotide (SSO) that selectively skips exon 27 of PBRM1 (Polybromo 1) pre-mRNA.

2. The composition according to Claim 1, wherein the splicing-switch oligonucleotide includes a sequence complementary to exon 27 of PBRM1 (Polybromo 1) pre-mRNA.

3. The composition according to Claim 2, wherein the splicing-switch oligonucleotide consists of the nucleotide sequence of SEQ ID NO: 1 or SEQ ID NO: 2.

4. The composition according to Claim 1, wherein the splicing-switch oligonucleotide selectively skips exon 27 by complementary binding to PBRM1 pre-mRNA, thereby inhibiting immune evasion mechanism in cancer cell.

5. A pharmaceutical composition for preventing or treating cancer comprising, as an effective component, a splicing-switch oligonucleotide (SSO) that selectively skips exon 27 of PBRM1 (Polybromo 1) pre-mRNA.

6. The pharmaceutical composition according to Claim 5, wherein the splicing-switch oligonucleotide includes a sequence complementary to exon 27 of PBRM1 (Polybromo 1) pre-mRNA.

7. The pharmaceutical composition according to Claim 6, wherein the splicing-switch oligonucleotide consists of the nucleotide sequence of SEQ ID NO: 1 or SEQ ID NO: 2.

8. The pharmaceutical composition according to Claim 5, wherein the effective component has one or both ends fused to a carrier, either directly or via a linker.

9. The pharmaceutical composition according to Claim 5, wherein it further includes a gene carrier besides the effective component.

10. The pharmaceutical composition according to Claim 5, wherein the cancer is any one selected from uterine corpus endometrial carcinoma (UCEC), bladder cancer (BLCA), rectal adenocarcinoma (READ), breast cancer (BRCA), prostate adenocarcinoma (PRAD), stomach adenocarcinoma (STAD), lung adenocarcinoma (LUAD), lung squamous cell carcinoma (LUSC), colon adenocarcinoma (COAD), kidney renal papillary cell carcinoma (KIRP), and liver hepatocellular carcinoma (LIHC).

11. A method for regulating expression of PBRM1 (Polybromo 1) protein in mammalian cells comprising transducing mammalian cells with a splicing-switch oligonucleotide (SSO) that selectively skips exon 27 of PBRM1 (Polybromo 1) pre-mRNA to induce splicing that selectively skips exon 27 of PBRM1 (Polybromo 1) pre-mRNA through complementary binding between the SSO and PBRM1 (Polybromo 1) pre-mRNA.
